# EUROPEAN PATENT APPLICATION

(11) **EP 3 056 245 A1**
(43) Date of publication of application: **17.08.2016**
(21) Application number: 16154334.3
(22) Date of filing: 04.02.2016
(51) Int. Cl.: A61N 5/10

(54) **RADIATION THERAPY GUIDED USING PET IMAGING**

(30) Priority: 11.02.2015 US 201562114682 P
(71) Applicant: Cubresa Inc., Winnipeg, Manitoba R3H 0N1 (CA)
(72) Inventor: Saunders, John, Winnipeg, Manitoba R3H 0N1 (CA); Schellenberg, James, Winnipeg, Manitoba R3H 0N1 (CA)
(74) Representative: Franke, Dirk

(57) **Abstract**

Radiation therapy of a lesion within a patient is guided to take into account movement of the lesion caused by respiration and/or cardiac effects by using MRI or other imaging system suitable for locating the lesion to image the patient while on the treatment support and using a PET detection system responsive to a radiation source preferentially taken up by the lesion and registered with the MRI so as to monitor movement of the lesion in real time and thus guide the beam of the RT.

## Description

This invention relates to a guided Radiation Therapy system using PET detection system in association with another imaging modality such as MRI (Magnetic Resonance Imaging) for location of a lesion for treatment and for controlling the radiation to the lesion.

### BACKGROUND OF THE INVENTION

An external beam radiotherapy device includes a means of generating a high energy radiation beam of electrons, X-Rays or gamma rays which are directed into the patient to kill cells in lesions such as tumours, arterial venous malformations etc. The source of high energy is mounted on a gantry which can rotate about an axis which is approximately parallel to the patient lying on the patient couch. The patient is treated using an electron beam, an X-Ray beam or a beam of gamma rays focused at a target by the combination of the use of collimators and the rotation of the beam. The patient is placed on a couch which can be positioned such that the target lesion can be located in the plane of the electron beam as the gantry rotates.

The objective of the radiation therapy is to target the lesion with a very high dose of radiation but to have minimal impact on all the surrounding normal tissue. It is critical that all the cells in the lesion be killed since any residual cells can multiply and reform the lesion and tumours for example often become more malignant and treatment resistant on regeneration. It is also critical to minimize collateral damage to adjacent tissue since quality of life post treatment is an important objective of any therapeutic regime. A critical task in the workflow is to precisely locate the position of the lesion in 3 dimensional space. The best technique for this is MRI since this technology provides excellent soft tissue - soft tissue contrast as well as soft tissue-hard tissue boundaries. Even though MRI provides good location of the lesion at the time of measurement, these images are recorded normally 1 to 3 days prior to the treatment and so may not be completely representative of the location of the lesion on the day of treatment. The oncologists therefore tend to increase the target volume to be certain that all of the lesion tissue receives sufficient dose to eliminate all lesion cells. This does produce collateral damage and may have a significant impact of the quality of life of the patient. Ideally, one would image the patient as they enter the radiation device for treatment to provide exact location of the lesion. In many radiation treatment units the patient is imaged by a CONE Beam CT just prior to the treatment. This has two functions namely to verify lesion location and to calculate the dose of radiation. Unfortunately, the cone beam CT while adequate for dosage determinations is not ideal for lesion location. Another complementary imaging modality at this time would be ideal. IMRIS in collaboration with Varian have attempted to resolve this issue by placing an MRI unit inside the radiation bunker at 180° to the radiation beam generator so that the patient on the couch is imaged and then the couch is rotated so that the patient on the couch then slides into the radiation unit. This does provide information on the position of the lesion with the patient in the orientation for treatment. This is an expensive method to verify lesion location and does significantly increase the procedure time. The normal procedure for radiation therapy is for the patient to come to the clinic daily for up to several weeks (4-6 weeks). This large number of treatments is a consequence of the lack of knowledge of lesion location. As the treatment proceeds the lesion may decrease in size, may move and the patient may lose weight. These all contribute to decreased accuracy of lesion location. It is not practical and would also be very expensive to image the patient before each radiation procedure so in practice it is not done.

This lack of exact knowledge of lesion location at the moment of treatment is bad enough but an even more serious challenge to accurate radiation treatment is the effect of lesion motion on the ability to precisely target the target lesion. Apart from the brain and some extremities most of the body is subject to either respiratory, cardiac or peristaltic motion or even a combination of these movements. This motion results in the lesion moving and so there is no guarantee that continuous targeting for treatment is possible. In a combined CT radiotherapy unit the main purpose of the CT is to locate the lesion but the oncologists always increases the volume of the radiation dose to attempt to kill all the tumor cells because CT does not give adequate soft tissue-soft tissue contrast. Fusing the CT with previously acquired MRI images can improve lesion location and the use of this combined modality can result in less collateral damage but cannot take into account any motion.

Ideally, one would use an imaging device which could rapidly take an image at the start of the treatment to focus the radiation beam and then use this or a complementary device to monitor the lesion in real time.

### SUMMARY OF THE INVENTION

The object of invention is to add nuclear imaging to the radiation system such that the position of the tumor in the body can always be known and this information can be transmitted rapidly to the controls directing the radiation beam.

According to the invention there is provided a method for radiation therapy of a patient comprising:
locating a patient on a patient support device, the patient having a lesion requiring radiation therapy;
while the patient is on the patient support device using a first imaging system to obtain a one or more first images of a location of the lesion within the patient;
while the patient is on the patient support device using a source of radiation therapy on a gantry to apply a controlled guided beam of radiation to the lesion by rotation of the source around an axis passing through the lesion;
applying to the patient a suitable radioisotope for PET imaging of gamma radiation emitted by the lesion;
providing a plurality of PET detectors lying in a circular (or elliptical, for example) array around the lesion for detecting the gamma emissions to generate a PET image;
during the application of the radiation therapy obtaining images of the lesion or a location on the body of the patient correlated with the lesion using the array of PET detectors responsive to the emitted gamma radiation so as to determine movement of the lesion which occurs during the radiation therapy;
registering the images of the lesion obtained by the PET detectors with said one or more images obtained by the imaging system;
and controlling the dose applied by the source of radiation in response to the movement of the lesion detected by the PET detectors.

Preferably the PET detectors are of sufficient size and in appropriate position to allow both ends of the coincident beams of gamma radiation to be detected at the same time.

Preferably the PET detectors are positioned so that the radiation beam from the source has unimpeded access to the patient at all times during gantry rotation.

Preferably the array of PET detectors substantially wholly surrounds the lesion of the patient omitting only the PET detectors over an area sufficient to allow penetration of the beam from the source.

Preferably the circular or elliptical array of PET detectors lies in a plane which is inclined relative to a radial plane of the axis of rotation of the source on the gantry.

Preferably the array of PET detectors is maintained stationary during PET imaging.

Preferably high resolution images in both the PET image and the first image are obtained using breath holding or respiratory gating.

Preferably the PET image is acquired by gating using the first or MRI imaging methodology and then the very high resolution PET image thus obtained is fused with the first or MRI images obtained with either respiratory gating or breath hold.

Preferably, in subsequent treatment procedures the PET image is acquired just prior to treatment to verify the size and location of the lesion relative to previous treatments.

Preferably at least one of the PET images is obtained simultaneously with the imaging by the first or MRI imaging system.

The registration of the PET and MRI images can be carried out geometrically by physical points on the imaging systems or on the patient support device or by image comparison techniques.

Preferably the control of the radiation therapy system is carried out in real time in response to real time images obtained by the PET imaging system so that it is desirable to maintain the PET detections system stationary during the procedure.

In some cases the control of the radiation therapy system is carried out by halting the dose whenever the lesion is detected to have moved beyond a predetermined allowable position.

In other cases the control of the radiation therapy system is carried out by controlling a focused position of a beam of the radiation therapy system in dependence on the movement of the lesion, wherein the beam is rotated around an axis and wherein the focused position is moved in a radial or an axial direction.

Where the first imaging system is MRI the magnet of the MRI is preferably moved away from the patient support device so as to allow the radiation therapy. In this case preferably the radiation source and the patient support device are located in a room shielded to prevent release of the radiation and the room includes a door through which a magnet moves to remove the magnet from the room during the therapy.

Where movement of the first imaging system is not carried out and the patient is moved preferably the patient is moved from the first imaging system to the radiation therapy system without moving the patient position relative to the patient support device.

Where the first imaging system is stationary and the patient is moved from the first imaging system to the radiation therapy system preferably the imaging coordinates are maintained by application of fiducials which are observable in all the multi-modality images

Preferably the PET imaging system operates in constant mode.

In some cases there is provided a marker on the patient and wherein a gamma imaging system monitors the movement of this marker to assist in controlling the guidance of a beam of the radiation therapy system in response to the movement of the body of the patient.

Preferably sets of images of the PET imaging system and the MRI imaging system are fused together such that the observed PET image demonstrates all the features of the first image of the first imaging system.

The invention will provide much better control of radiation treatment of tumors located in regions of the body subject to motion. It will result in a bigger cell kill per grey of irradiation. It will minimize the irradiation of normal tissue adjacent to the tumor lesion and hence collateral damage.

Positron emission tomography (PET) is a functional imaging technique that produces a three-dimensional image of functional processes in the body. The system detects pairs of gamma rays emitted indirectly by a positron-emitting radionuclide (tracer), which is introduced into the body on a biologically active molecule. Three-dimensional images of tracer concentration within the body are then constructed by computer analysis.

If the biologically active molecule chosen for PET is fludeoxyglucose (FDG), an analogue of glucose, the concentrations of tracer imaged will indicate tissue metabolic activity by virtue of the regional glucose uptake. Use of this tracer to explore the possibility of cancer including metastatic cancer is the most common type of PET scan in standard medical care (90% of current scans). However, on a minority but ever increasing basis, many other radioactive tracers are used in PET to image the tissue concentration of many other types of molecules of interest.

The patient is injected with a tumor targeting radioactive substance for detection by the PET detection system. The choice of radioactive compound is guided by the type of lesion to be irradiated. The choice of marker molecule depends on the characteristics of the target to be identified. One chooses a marker that will be attracted preferentially to the target relative to other tissue in the body. As the "PET" radioisotope undergoes positron emission decay, it emits a positron, an antiparticle of the electron with opposite charge. The emitted positron travels in tissue for a short distance (typically less than 1 mm), during which time it loses kinetic energy, until it decelerates to a point where it can interact with an electron. The encounter annihilates both electron and positron, producing a pair of annihilation (gamma) photons moving in approximately opposite directions. The most significant fraction of electron-positron annihilations results in two 511 keV gamma photons being emitted at almost 180 degrees to each other; hence, it is possible to localize their source along a straight line of coincidence. These are detected when they reach a scintillator in the scanning device. The PET detection system must include two detectors to detect the photons 180 degrees to one another.

### BRIEF DESCRIPTION OF THE DRAWINGS

One embodiment of the invention will now be described in conjunction with the accompanying drawings in which:
Figure 1 is an isometric view of a patient in an MRI scanner with a PET detection system located for imaging radioactive compound concentrated in a tumour according to the present invention.
Figure 2 is an isometric view of a radiation therapy unit including the PET detection system to image the radioactive compound concentrated in a neck tumour.
Figure 3 is an enlarged end view of the therapy system and PET detection system.
Figure 4 is a side elevational view of the therapy system PET detection system.
Figure 5 is a side elevational view of a second embodiment of the therapy system PET detection system according to the present invention where the PET detection ring entirely surrounds the patient and the radiation treatment source is cyclically halted during each rotation of the gantry to avoid intersecting the PET ring. Also this embodiment shows an arrangement in which the patient support can be tilted.

### DETAILED DESCRIPTION

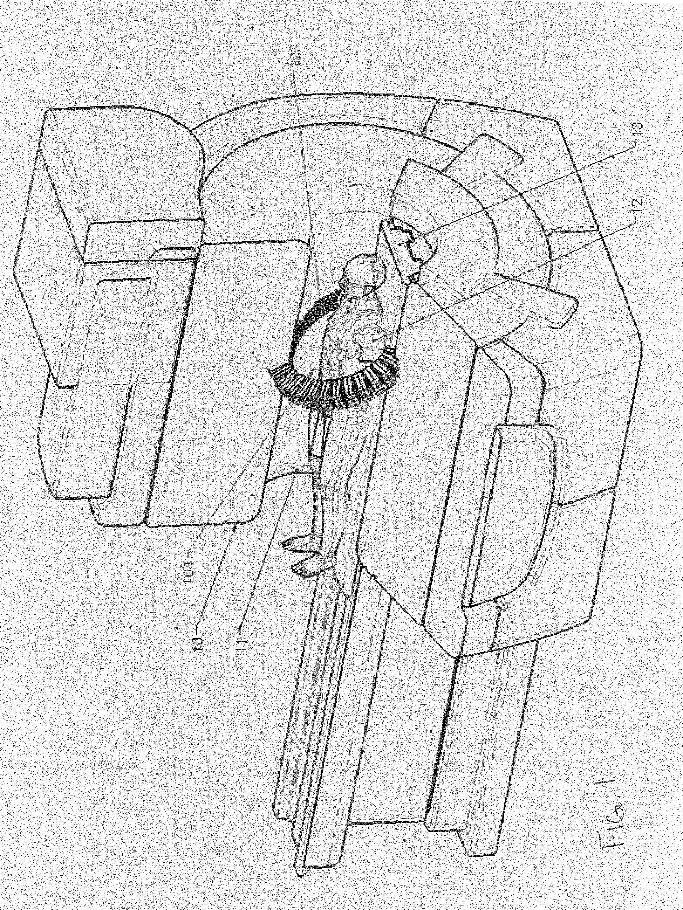

In Figure 1 is shown schematically a magnetic resonance imaging system which includes a magnet 10 having a bore 11 into which a patient 12 can be received on a patient table 13. This patient table is in fact a component of the patient support system and this is moved with the patient in the identical position on the patient table as the patient moves from imaging device to treatment device. The system further includes an RF transmit body coil 10A which generates a RF field within the bore. In one embodiment the magnet is movable being carried on a rail system (not shown) with a support suspended on the rail system. In another embodiment, the magnet is stationary and the patient enters the magnet on a table top which can also enter the radiation therapy unit so that the patient is in the same position for both imaging and treatment. In another embodiment the patient, the magnet is stationary and the patient is placed on an MRI only patient table and in this case co-registration is obtained by using fiducials which are observable in all forms of imaging to be undertaken.

The system further includes a receive coil system 10B which is located at the iso-center within the bore and receives signals generated from the human body in conventional manner. A RF control system 10C acts to control the transmit body coil and to receive the signals from the receive coil. The PET detection system 120 comprises a ring of PET detectors 125. The same arrangement is employed if a patient is imaged outside the treatment room and again, the patient's position relative to the patient support system must remain constant throughout.

As shown, the PET detection system 120 can enter the magnet and can have MRI visible fiducials such that the PET detection system 120 has a position which is registered to the anatomical images of the patient acquired by the MRI system.

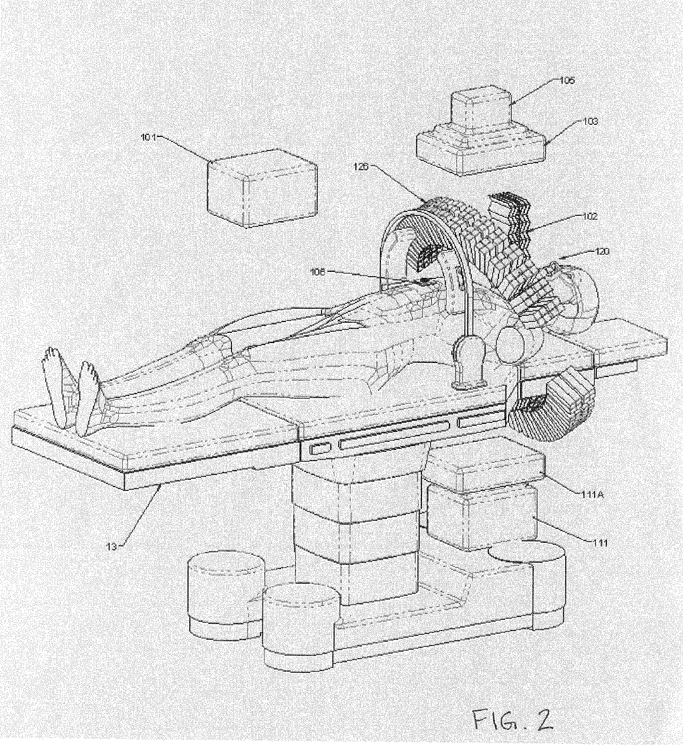

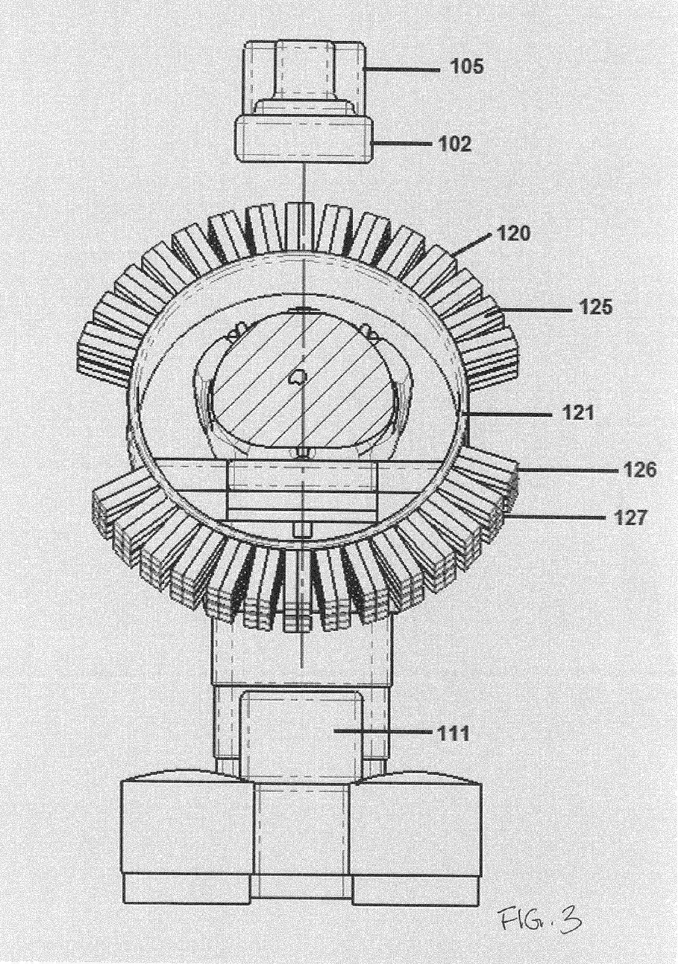

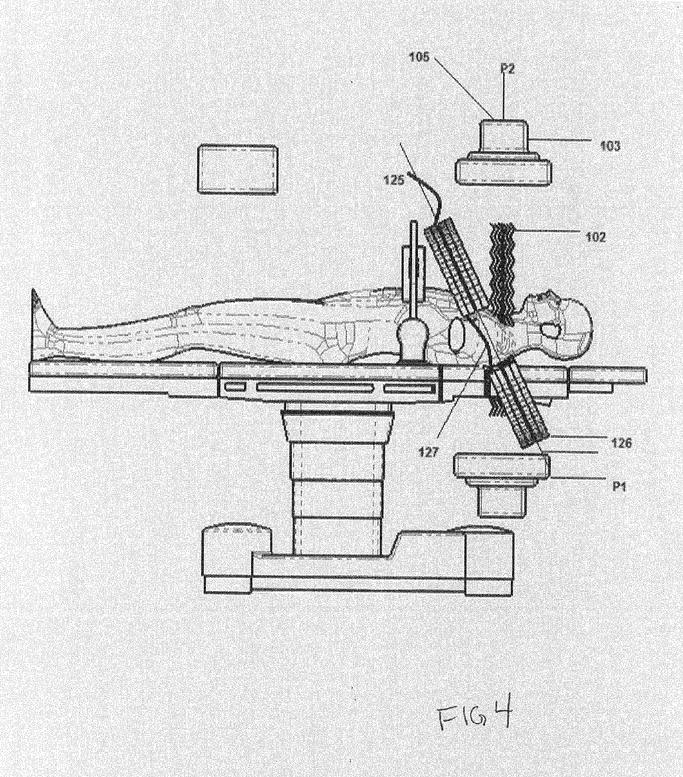

The MRI system is used in conjunction with a patient radiation therapy system shown better in Figure 2 with the magnet 10 of the MRI system removed or the patient moved from the MRI system to the radiation therapy system on the same couch top. Figures 3 and 4 provide perpendicular and expanded views of the patient illustrating the location, support and shielding of the PET detection system 120. The therapy system includes a bunker or room within which is mounted the patient support 13 and a radiation gantry 105. The gantry carries a radiation source 103, which is in most cases a linear accelerator associated with a collimator for generating a beam 102 of radiation. Systems are available for example from Varian where the radiation system and the patient support are controlled to focus the beam onto any lesion of any shape within the patient body, bearing in mind complex shapes of lesion which are required to be radiated.

The patient having a lesion requiring radiation therapy is placed on the treatment support 13 and prepared for the radiation therapy on the treatment support.

During the initial imaging phase, the magnet of the MRI system is carried into the imaging position at the treatment support for imaging the patient while on the treatment support. If a stationary magnet is employed the patient is moved into the magnet of the MRI unit. In the movable magnet scenario, the magnet of the MRI system is then moved away from the treatment support through a door of the bunker on the rails so as to allow the radiation therapy to commence. In the stationary magnet concept, the patient is removed from the magnet and in one embodiment is placed on a trolley and wheeled to the treatment room. In another embodiment, the patient is transferred to the patient support device in the treatment bunker with additional fiducials used to co-register the images from different modalities. Thus the patient is placed on the support or couch which can move such that the electron beam always irradiates the target volume. The gantry rotates such that the focus of the beam is always a relatively small volume. The table 13 can move in three directions and this combined with the rotation focuses the treatment within a specified volume which is arranged to be as close as possible to the margins of the lesion in the patient. The goal is that this volume is the target lesion and only the target lesion. It is normally required that the entire target lesion receives the same maximum dose of radiation so that all cells within the targeted volume die. In certain cases, prostate treatment for example, it may be desired to increase the dose to some regions of the gland relative to others and this is guided by using the PET images or PET images fused with MRI images. It is required that damage to adjacent normal tissue be minimal.

The radiation control unit 111 includes an electrical interface 111A which allows control over its radiation beam over location and time. There is provided a boom system to allow both the radiation unit to be moved sufficiently far from the magnet and moved into position for the radiation therapy.

A system is provided to generate a correlation between the coordinates systems of the patient that is the patient support table, the MR images, the gamma images and the RT beam 102. The latter can be decomposed into the physical location of the radiation therapy unit relative to the patient support table, and the beam coordinate system relative to the radiation therapy unit.

The radiation beam as the gantry rotates is cylindrical in shape as shown in Figure 1. The radiation beam is able to operate in either pulsed or constant mode. When in pulsed mode there will be coordination between the radiation beam and the detection beam such that only one is in operation at any time. In one embodiment of the patent there is a radioactive marker on the chest of the patient and gamma camera detection system monitors the movement of this marker. The gamma camera detection system provides a three dimensional representation of the marker movement. There are metal (lead or tungsten for example) boxes which cover the PET and gamma camera detection systems and will lead to the marker to attenuate significantly any scatter radiation from the high power radiation therapy. There is a metal (lead or tungsten for example) shield which shields the marker from the patient and more importantly the patient from the marker. This is shown in Figure 2. The frequency of this marker (different radioactive isotope) is different than that used to measure the location of the lesion. During the training session, the typical location of the lesion relative to the chest marker will be determined so that this information can guide the PET detection system during radiation therapy. In one embodiment of the patent, this idea of a respiratory marker to guide the radiation therapy will be the unique image guidance in the therapy vault. The training will correlate the position of the lesion with the position of the radioactive marker. The emission of gamma rays from the patient could be masked by the very high energy radiation of the beam. The high energy beam will be directed at the lesion but there will be scatter and some of this scattered radiation will enter either the PET or gamma camera detection systems unless filters are applied using software filtering. The attenuation of scattered radiation is minimized by the appropriate use of metal shields (lead or tungsten for example) of appropriate thickness. The PET images from the lesion guide the treatment in one of two ways. In the first, the radiation is turned off when the lesion moves away from the designated killing zone as detected by the imager. In the second, real time images are transmitted to the radiation device to modify the direction of the beam such that it is always on target.

The PET detection system must be located such that the coincident beams can be detected at the same time. The normal commercial PET device has a ring of detectors which completely encircle the patient and the PET technology detects the coincident rays. However, this is not possible if the PET images are to be recorded during irradiation by the high energy radiation beam and so the PET detector system is constructed to have a gap in the detectors to allow the radiation beam unimpeded access to the patient at all times as the gantry rotates.

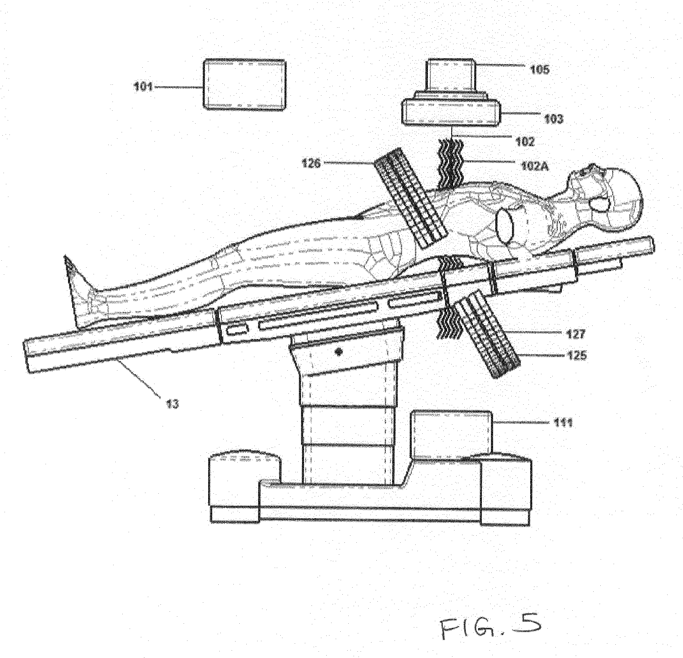

An alternative embodiment shown in Figure 5 uses an arrangement in which the radiation source 103 is turned off when the gantry 105 is in the position where the beam 102 would irradiate the completely circular ring 126 of PET detectors 125. This requires a special radiation treatment program controlled by the control unit 111 to take into account that rotation of the source is not a complete circle.

Rotation and movement of the PET detection ring in the axial direction is be permitted since in our invention the PET detection ring must measure the radiation in real time and hence must be maintained stationary. The detectors are of sufficient size and in appropriate position to allow both ends of the coincident beams to be detected at the same time. The PET detectors are positioned so that the radiation beam has unimpeded access to the patient at all times during gantry rotation. A typical example is shown particularly in Figure 4.

It is known that on average the best tumor delineation within a human body is obtained using MRI. In one embodiment of the invention, prior to the initial radiation, the patient will have an MRI image followed immediately by a PET image depending on the isotope to be injected. The two sets of images are co-registered using mechanical registration methodology. The two sets of images could be acquired simultaneously. The sets of images are fused together such that the observed PET image will demonstrate all the features of the MRI image. High resolution images in both modalities are obtained using breath holding or respiratory gating. The effect of motion is detected using both modalities and where useful the PET image is corrected by gating it to the MRI image and then fusing these results into the PET image. In subsequent treatment procedures the PET image is acquired just prior to treatment to verify the size and location of the lesion relative to previous treatments. In this way the invention guides the radiation treatment to take into account any lesion shrinkage from treatment to treatment and also to take into account any physical changes in the patient.

The arrangement described above and shown in the drawings provides a method for radiation therapy of a patient where the patient having a lesion requiring radiation therapy is located on a patient support device 13. While the patient is on the patient support device 13 a first MR imaging system including magnet 10 is used to obtain a one or more first images of a location of the lesion within the patient. The patient may receive the initial images while lying on an imaging table (eg MRI) and then be transferred to the radiation patient support device with image co-registration being obtained using appropriate fiducials. While the patient is on the patient support device a source 103 of a beam 102 of radiation therapy on a gantry 105 is used to apply a controlled guided beam 103 of radiation to the lesion by rotation of the source around an axis passing through the lesion.

A suitable radioisotope for PET imaging of gamma radiation emitted by the lesion is applied to the patient and the emitted pairs of gamma rays are detected by a plurality of PET detectors 125 lying in a circular or elliptical array 126 around the lesion for detecting the gamma emissions to generate a PET image. During the application of the radiation therapy, images of the lesion or a location on the body of the patient correlated with the lesion are obtained using the array of PET detectors responsive to the emitted gamma radiation so as to determine movement of the lesion which occurs during the radiation therapy.

The images of the lesion obtained by the PET detectors are registered with the images obtained by the MR imaging system in the control system 111 which acts to control the dose applied by the source of radiation in response to the movement of the lesion detected by the PET detectors.

The PET detectors 125 are of sufficient size and in appropriate position to allow both ends of the coincident beams of gamma radiation to be detected at the same time. In one embodiment of the system the PET detectors 125 are positioned so that the radiation beam from the source has unimpeded access to the patient at all times during gantry rotation. Thus the array 126 of PET detectors substantially wholly surrounds the lesion of the patient omitting at spaces 127 only the PET detectors over an area sufficient to allow penetration of the beam from the source as shown in Figure 4. The circular array of PET detectors lies in a plane P1 which is inclined relative to a radial plane P2 of the axis of rotation of the source on the gantry. In this way as the beam rotates with the gantry in the plane P2, the beam passes through the two spaces in the ring of detectors on either side of the patient support table with the spaces being only large enough to allow the beam to pass without impingement. This allows the array of PET detectors to be maintained stationary during PET imaging to obtain a real time image of the movement of the lesion to control the beam operation.

As shown in Figure 5, the patient support device 13 is elevated at the head end so that the angle of the beam relative to the table top and hence the patient varies from the conventional 90°. This of course also changes the angle of the patient and the lesion relative to the PET array 126. The incident angle of the high energy beam relative to the beam of gamma rays from the radioactive substance embedded in the lesion can thus be modified to provide optimal imaging of the lesion. Software methods calculate the ideal angles to obtain optimal image performance. They also provide the best methods to detect lesion moving in and out of the irradiation zone so that radiation can be guided precisely.

An alternative embodiment shown in Figure 5 to achieve the same objective is to have PET detectors complete the circle and to turn the high energy radiation off when rotation of the gantry would bring the high energy radiation beam into collision with the gamma ray beam. This requires programming of the high energy beam protocol of the lesion to maximize irradiation of the lesion with this less than 360 degrees irradiating path.

The arrangement as described herein therefore provides the PET detection system 120 including the ring 126 and software control 101 to add PET imaging to the radiation system such that the position of the lesion in the body can always be known and this information can be transmitted rapidly to the control system 111 directing the radiation beam. The patient is injected with a tumor targeting radioactive substance such F-18 fluorodeoxy glucose or similar radioactive compounds. The choice of radioactive compound is guided by the type of lesion to be irradiated.

The radiation beam can either be pulsed or constant. This allows the imaging by the PET detection system to be sequential to the beam (milliseconds to seconds alternation) or simultaneous with the beam. The radiation beam can operate in either pulsed or constant mode. When in pulsed mode there is coordination between the radiation beam and the detection beam such that only one is in operation at any time.

In one embodiment there is a radioactive marker 106 on the chest of the patient and so that there is provided gamma camera detection device 121 which monitors the movement of this marker. The gamma camera detection device provides a three dimensional representation of the marker movement.

The radiation frequency of this marker is obviously different since a different radioactive isotope and a different energy device is used to measure the location of the lesion. During a training session, the typical location of the lesion relative to the chest marker is determined so that this information can guide the PET detection system during radiation therapy.

In one embodiment of the patent, the use of this respiratory marker 106 to guide the radiation therapy provides unique image guidance in the therapy vault. The training will correlate the position of the lesion with the position of the radioactive marker 106 so that the position only of the latter can be detected in the RT and used to guide the RT beam. The PET images from the lesion can guide the treatment in one of two ways. In the first, the radiation is simply turned off when the lesion moves away from the designated killing zone as detected by the imager. In the second, real time images are transmitted to the radiation device to modify the direction of the beam such that it is always on target. The direction can be controlled by the gantry in a radial direction R to change the radial position of the focus F. Alternatively or in addition, software control of collimators can change the intensity of the beam either radially or axially to change the axial and radial positions of the focus.

## Claims

1. A method for radiation therapy of a patient comprising:
locating a patient on a patient support device, the patient having a lesion requiring radiation therapy;
while the patient is on the patient support device using a first imaging system to obtain one or more first images of a location of the lesion within the patient;
while the patient is on the patient support device using a source of radiation therapy on a gantry to apply a controlled guided beam of radiation to the lesion by rotation of the source around an axis passing through the lesion;
applying to the patient a suitable radioisotope for PET imaging of gamma radiation emitted by the lesion;
providing a plurality of PET detectors lying in a circular array around the lesion for detecting the gamma emissions to generate a PET image;
during the application of the radiation therapy obtaining images of the lesion or a location on the body of the patient correlated with the lesion using the array of PET detectors responsive to the emitted gamma radiation so as to determine movement of the lesion which occurs during the radiation therapy;
registering the images of the lesion obtained by the PET detectors with said one or more images obtained by the imaging system;
and controlling the dose applied by the source of radiation in response to the movement of the lesion detected by the PET detectors.

2. The method according to claim 1 wherein the PET detectors are of sufficient size and in appropriate position to allow both ends of the coincident beams of gamma radiation to be detected at the same time.

3. The method according to claim 1 or 2 wherein the PET detectors are positioned so that the radiation beam from the source has unimpeded access to the patient at all times during gantry rotation.

4. The method according to any one of claims 1 to 3 wherein the array of PET detectors substantially wholly surrounds the lesion of the patient omitting only the PET detectors over an area sufficient to allow penetration of the beam from the source.

5. The method according to any one of claims 1 to 4 wherein the circular array of PET detectors lies in a plane which is inclined relative to a radial plane of the axis of rotation of the source on the gantry.

6. The method according to any one of claims 1 to 5 wherein the array of PET detectors is maintained stationary during PET imaging.

7. The method according to any one of claims 1 to 6 wherein the PET detectors form a complete circle wherein the radiation source is off when rotation of the gantry would bring the high energy radiation beam into collision with the PET detectors.

8. The method according to any one of claims 1 to 7 wherein, in subsequent treatment procedures, the PET image is acquired just prior to treatment to verify the size and location of the lesion relative to previous treatments.

9. The method according to any one of claims 1 to 8 wherein at least one of the PET images is obtained simultaneously with the imaging by the first imaging system and the control of the radiation therapy system is carried out in real time in response to real time images obtained by the PET imaging system.

10. The method according to any one of claims 1 to 9 wherein the control of the radiation therapy system is carried out by halting the dose whenever the lesion is detected to have moved beyond a predetermined allowable position.

11. The method according to any one of claims 1 to 10 wherein the control of the radiation therapy system is carried out by controlling a focused position of a beam of the radiation therapy system in dependence on the movement of the lesion, wherein the beam is rotated around an axis and wherein the focused position is moved in a radial or an axial direction.

12. The method according to any one of claims 1 to 11 wherein there is provided a marker on the patient and wherein gamma camera imaging system monitors the movement of this marker to assist in controlling the guidance of a beam of the radiation therapy system in response to the movement of the body of the patient.

13. The method according to any one of claims 1 to 12 including moving the patient from the first imaging system to the radiation therapy system with moving the patient position relative to the patient support device but using multi-imaging modality markers to co-register all images to the patient.

14. The method according to any one of claims 1 to 13 including elevating the patient support device at the head end so that the angle of the beam relative to the table top and hence the patient is variable from the conventional 90°.

15. The method according to any one of claims 1 to 14 wherein the incident angle of the high energy beam relative to the beam of gamma rays from the radioactive substance embedded in the lesion is varied to provide optimal imaging of the lesion.
